(19) Europäisches Patentamt
European Patent Office
Office européen des brevets

(11) Publication number: **0 216 511 B1**

(12) **EUROPEAN PATENT SPECIFICATION**

(45) Date of publication of patent specification: **27.10.93** (51) Int. Cl.$^5$: **A61K 31/70**, A61K 31/505

(21) Application number: **86306495.2**

(22) Date of filing: **21.08.86**

(54) **Inhibition of in vitro infectivity and cytopathic effect of HTLV-III/LAV by 2'3'-dideoxycytidine.**

(30) Priority: **26.08.85 US 769017**

(43) Date of publication of application:
**01.04.87 Bulletin 87/14**

(45) Publication of the grant of the patent:
**27.10.93 Bulletin 93/43**

(84) Designated Contracting States:
**AT BE CH DE FR GB IT LI LU NL SE**

(56) References cited:
**EP-A- 0 206 497**

**JOURNAL OF CELLULAR PHYSIOLOGY, vol. 121, no. 2, November 1984, pages 402-408, Alan R. Liss, Inc.; M.A. WAOAR et al.: "Effects of 2',3'-dideoxynucleosides on mammalian cells and viruses"**

**CANCER LETTERS, vol. 8, 1980, pages 307-315, Elsevier/North-Holland Scientific Publishers Ltd; P. FURMANSKI et al.: "Inhibition by 2',3'-dideoxythymidine of retroviral infection of mouse and human cells"**

**PROC. NATL. ACAD. SCI., USA, vol. 83, March 1986, pages 1911-1915; H. MITSUYA et al.: "Inhibition of the in vitro infectivity and cytopathic effect of human T-lymphotrophic virus type III/lymphadenopathy-associated virus (HTLV-III/LAV) by 2',3'-dideoxynucleosides"**

(73) Proprietor: **THE UNITED STATES OF AMERICA as represented by the Secretary, United States Department of Commerce
National Technical Information Service,
Office of Government Inventions and Patents,
5285 Port Royal Road
Springfield, Virginia 22161(US)**

(72) Inventor: **Mitsuya, Hiroaki
259 Congressional Lane T-2
Rockville Maryland 20852(JP)**
Inventor: **Broder, Samuel
6004 Rossmore Drive
Bethesda Maryland 20814(JP)**

(74) Representative: **Jump, Timothy John Simon et al
F.J. Cleveland and Company
40-43 Chancery Lane
London WC2A 1JO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to the inhibition of in vitro infectivity and the cytopathic effect of HTLV-III/LAV by 2',3'-dideoxycytidine and the mono- or triphosphates thereof.

Since 1983 it has developed that the virus HTLV-III is the etiologic agent of acquired immunodeficiency syndrome (AIDS) and related diseases. Medicine is currently endeavoring to find agents which will check the ravages of the disease. In Science (1983) Vol 220, P506-508 Montagnier discloses the investigation and isolation of an agent subsequently defined as HTLV-III. In this connection, a number of patent applications have been filed naming as a co-inventor Robert C. Gallo; one patent has issued, U.S. Patent No. 4,520,113, which mainly relates to the detection of antibodies in the sera of AIDS and PRE-AIDS patients.

The present invention, however, lies in a method of alleviating the effects of HTLV-III by the in vitro contacting of the virus with a particular pyrimidine base, namely, 2',3'-dideorycytidine and the related mono- and tri-phosphates of this base.

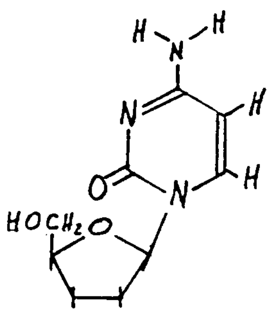

2',3'-dideoxycytidine

The following references are relevant to determining the state of the art prior to the present invention:

Furmanski, et al, "Inhibition by 2',3'-Dideoxythymidine of Retroviral Infection of Mouse and Human Cells," Cancer Letters, 8:307-315, 1980;

Wagar, et al, "Effects of 2',3'-Dideoxynucleosides on Mammalian Cells and Viruses, " Journal of Cellular Physiology, 121:402-408 (1984);

Rosen, et al, "The Location of Cis-Acting Regulatory Sequences in the Human T Cell Lymphotropic Virus Type III (HTLV-III/LAV) Long Terminal Repeat," Cell, 41:813-823, July 1985;

Time, August 12, 1985, p. 42 (column 3).

US Patent No. 4,434,788 to Nakataugawa shows the utilization of 3'-deoxyguanosine and 3'-deoxyuridine as anti-tumor agents; and

US Patent No. 4,081,534 to Elion et al shows various purine derivatives utilized against HSV.

The uniqueness of the HTLV-III virus and its resistance to treatment by known antiviral agents has been stated in Time magazine as follows: "Research conducted by Harvard's Haseltine and published in the July issue of the journal Cell [Cell, 41:813-823, 1985] reveals that the virus has a unique genetic component that allows it to reproduce itself a thousand times as fast as any other kind of virus. The mechanism for this reproduction is 'one of the biggest effects I've seen in biology,' says Haseltine. 'It helps explain why AIDS is such a devastating disease and why it can spread so fast.' In the process of rampant replication, the AIDS virus destroys its home, the T cell. Thus it is a peculiar feature of this disease that as it progresses, the helper T cells disappear and so does the virus. By then, however, the patient is invariably beyond recovery."

It has been noted that the chronicity of infection and the propensity of the virus to infect the brain make it necessary to develop new classes of remedies and drugs which have the potential for oral administration and penetration across the blood-brain barrier. In this study, the capacity of purine and pyrimidine nucleoside derivatives to inhibit the infectivity and cytopathic effect of HTLV-III/LAV in vitro were explored. With the ribose moiety of the molecule in a 2',3'-dideoxy-configuration, the pyrimidine (cytidine and thymidine) nucleosides tested suppressed the virus, although the thymidine derivative seemed to have substantially less activity. In general, it was observed that complete suppression of the virus occurs at doses that were 10- to 20-fold lower than those needed to inhibit the proliferation of the target T-cells and the immune reactivity of normal T-cells in vitro. An anlaysis of five adenosine congeners, which differed only in the sugar moiety, revealed that reduction (an absence of hydroxyl determinants) at both the 2' and

3' carbons of the ribose was necessary for an anti-viral effect, and an additional reduction at the 5' carbon nullified the anti-viral activity.

Accordingly the present invention provides in one aspect the use in the preparation of a medicament for the treatment of AIDS or a related disease of which HTLV-III is the etiological agent, of a therapeutically effective amount of 2',3'-dideoxycytidine or a mono- or triphosphate thereof, said therapeutically effective amount being insufficient to inhibit proliferation or immune reactivity of normal T-cells.

Figure 1-a shows the cytopathic effect of target T-cells.

Figures 1-b through 1-e illustrate the potent protective effect of 2',3'-dideoxyucleosides on the survival and growth of ATH8 cells when exposed to HTLV-III/LAV.

Figure 2A also illustrates the potent protective effect of 2',3-dideoxynucleosides on the survival and growth of ATH8 cells when exposed to HTLV-III/LAV.

Figure 2B illustrates the expression of the HTLV-III/LAV p24 gag protein in H9 cells.

Figure 2C shows the protection of ATH8 cells by adenosine congeners against the cytopathic effect of HTLV-III/LAV.

In Figure 1, which shows the survival and growth of ATH8 cells in the presence of 2',3'-dideoxynucleosides when exposed to HTLV-III/LAV, $2x10^5$ ATH8 cells were treated with polybrene, exposed to HTLV-$III_B$ (2,000 virus particles/cell), resuspended in culture tubes, and cultured (solid symbols) in the (a) absence or presence of (b) 50$\mu$M 2',3'-dideoxyadenosine (Calbiochem-Behring Corp., LaJolla, CA), (c) 50 $\mu$M 2',3'-dideoxyinosine (Calbiochem-Behring Corp.), (d) 1$\mu$ M 2',3'-dideoxycytidine (Calbiochem-Behring Corp.), and (e) 500 $\mu$M 2',3'-dideoxythymidine (P.L. Biochemicals Inc., Milwaukee, Wis.). Control cells were similarly treated but were not exposed to the virus (open symbols). At various time points, total viable cells were counted as described in Example 1.

In Figure 2A, which shows the inhibition of cytopathic effect of HTLV-III/LAV by 2',3'-dideoxynucleosides against ATH8 cells, $2x10^5$ ATH8 cells were preexposed to polybrene, exposed to HTLV-$III_B$ - (2,000 virus particles/cell) in culture tubes (solid columns) in the presence or absence of various concentrations of 2',3'-dideoxyadenosine, 2,'3'-dideoxyinosine, 2',3'-dideoxyguanosine (P.L. Biochemicals Inc.), 2',3'-dideoxycytidine, or 2',3'-dideoxythymidine. Control cells (open columns) were similarly treated but were not exposed to the virus. On day 5, total viable cells were counted as described in Example 1.

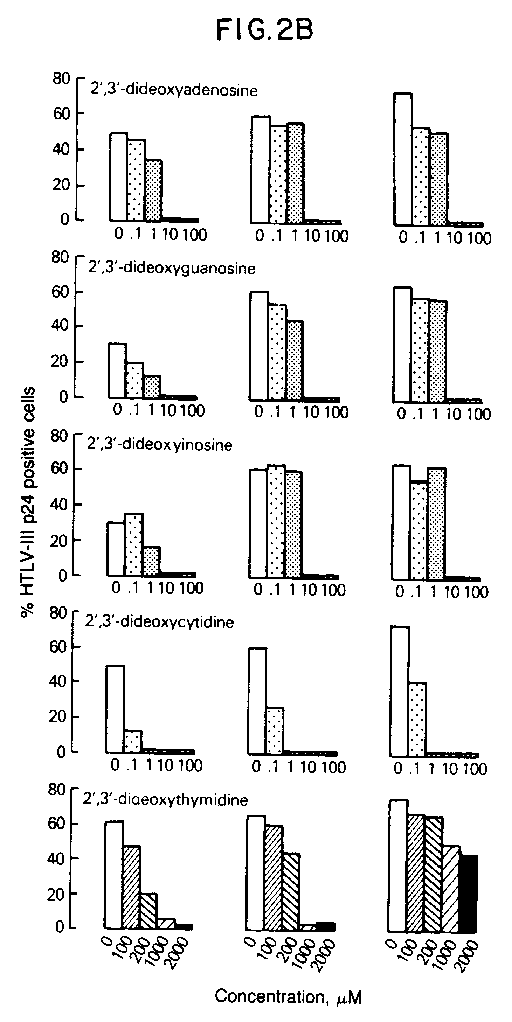

In Figure 2B, which shows the inhibition of the infectivity and replication of HTLV-III/LAV in H9 cells by 2',3'-dideoxynucleosides, $10^5$ H9 cells were exposed to various concentrations of 2',3'-dideoxynucleosides for 4 hours, then to 2 $\mu$ g/ml polybrene for 30 minutes, pelleted, and exposed to HTLV-$III_B$ (3,000 virus particles/cell) for 1.5 hours. Cells were resuspended in fresh complete medium and cultured in tubes at 37°C in 5% $CO_2$-containing humidified air. The cells were continuously exposed to 2',3'-dideoxynucleosides. On days 8 (left), 9 (middle), and 10 (right) in culture, the percentage of the target H9 cells expressing p24 gag protein of HTLV-III/LAV was determined by indirect immunofluorescence microscopy by using anti-HTLV-III/LAV p24 murine monoclonal antibody (M26 provided by Drs. F.D. Veronese and R.C. Gallo).

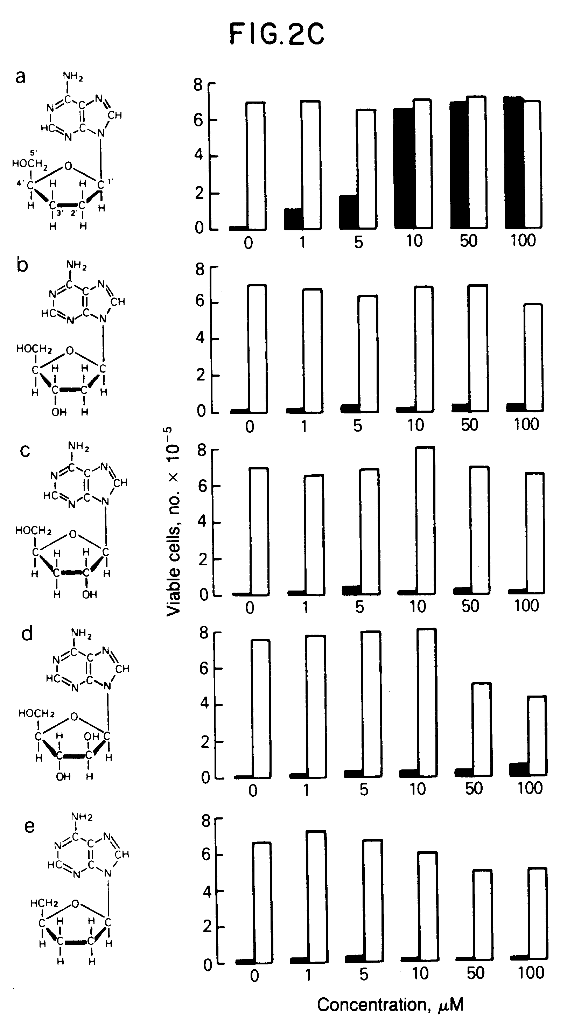

In Figure 2C, which shows protection of ATH8 cells by adenosine congeners against the cytopathic effect of HTLV-III/LAV, $2x10^5$ ATH8 cells were pre-exposed to polybrene, exposed to HTLV-$III_B$ (2,000 virus particles/cell), resuspended in culture tubes (solid columns) in the presence or absence of various amounts of adenosine congeners: (a) 2',3'-dideoxyadenosine, (b) 2'-deoxyadenosine (Calbiochem-Behring Corp.), (c) 3'-deoxyadenosine (cordycepin; Behringer-Mannheim GmbH, West Germany), (d) adenosine arabinoside and (e) 2',3',5'-trideoxyadenosine (both provided by Drs. D. Johns, J. Driscoll and G. Milne). The primed numbers in (a) refer to positions in the sugar moiety. Control cells (open columns) were not exposed to the virus. On day 5, the total viable cells were counted as described in Example 1.

In the system, HTLV-III/LAV (as cell-free virus) exerts a profound cytopathic effect on the target T-cells by day 4 in culture. By day 10, 98% of the cells are killed by the virus (Figure 1-a). The killing of cells can be monitored quantitatively as a function of the starting dose of virus (Table 1). When ATH8 cells are used in a 7-day assay, 5 virus particles/cell represented the minimum cytopathic dose of virus. In the experiments reported below, 2,000 or 3,000 virus particles/cell were used in order to test various compounds under conditions of substantial virus excess.

Figure 1 (b-e) and Figure 2A illustrate the potent protective effect of 2',3'-dideoxynucleosides on the survival and growth of ATH8 cells when exposed to HTLV-III/LAV. It has been found that concentrations greater than 0.5 $\mu$M of 2',3'-dideoxycytidine completely protected ATH8 cells and enabled them to survive and grow. This compound exhibited a strong anti-viral effect at doses that were 10- to 20-fold lower than those that inhibited growth of the target cells when no virus was present. In the present experimental conditions, 2',3'-dideoxythymidine required relatively high concentrations to exert a protective effect, and unlike the other comparable dideoxynucleosides tested, its capacity to nullify the cytopathic effect of the

virus was lost on day 10 of the culture (Figure 1-e and bottom of Figure 2A).

These anti-viral effects were confirmed in a different system, using the expression of the HTLV-III/LAV p24 gag protein in H9 cells (Figure 2B). The H9 cells are relatively resistant to the cytopathic effect of HTLV-III/LAV, and p24 gag protein expression following exposure to HTLV-III/LAV virions may be used as an index of viral infectivity and replication in vitro. The 2',3'-dideoxy-derivatives of cytidine were potent inhibitors of viral replication in the system, while 2',3'-didoexythymidine required relatively higher concentrations to mediate an anti-viral effect, and this compound allowed a resumption of viral replication by day 10 of culture (Figure 2B, bottom).

Also tested were the effects of the various 2',3'-dideoxynucleosides on the antigen-specific and lectin-induced reactivity of normal lymphocytes in vitro (Table 2). A normal clone (TM3) of tetanus-toxoid specific helper/inducer T-cells was used to monitor the effects of the compounds on antigen-driven activation, and normal circulating lymphocytes were used to monitor effects on pokeweed mitogen and phytohaemagglutinin-driven activation. Concentrations of these compounds, including those that were 10- to 20-fold higher than those necessary to block the in vitro infectivity and cytopathic effect of HTLV-III/LAV left the in vitro immune reactivity of normal lymphocytes basically intact.

The mechanisms by which 2',3'-dideoxynucleosides suppress the replication of HTLV-III/LAV are not known. It is known that the 5'-triphosphate product of 2',3'-dideoxycytidine, and -dideoxythymidine can inhibit cellular DNA polymerases beta and gamma, as well as viral reverse transcriptase, but not mammalian DNA polymerase alpha. DNA polymerase alpha is assumed to be the key DNA synthetic enzyme for DNA replication during cell division, and it also has a role in DNA repair. Of interest, Herpes simplex type I DNA polymerase is reported to be as resistant to 2',3'-dideoxythymidine as cellular DNA polymerase alpha. Unphosphorylated dideoxynucleosides have rather negligible effects on the growth of cultured mammalian cells (a phenomenon which is confirmed here in human T-cells). This is believed to be so because of inefficient intracellular conversion to the corresponding 5'-triphosphates coupled with the resistance of DNA polymerase alpha to low levels of the 5'-triphosphates.

Example 1

With reference to Table 1, a tetanus toxoidspecific T-cell line, established by repeated cycles of stimulation with antigen as described in Mitsuya, et al, Science, 225:1484-1486 (1984), was cloned in the presence of lethally irradiated (12,000 rad) human T-lymphotropic virus type I (HTLV-I)-producing MJ-tumor cells in 96-well microtiter culture plates (Costar, Cambridge, MA). Clone ATH8 (obtained from a culture plated at 0.5 cells per well) was selected for drug screening on the basis of its rapid growth (in the presence of interleukin-2) and exquisite sensitivity to the cytopathic effect of HTLV-III/LAV. ATH8 clone bears several distinct copies of HTLV-I in its genome when assessed by Southern blot hybridization using a radiolabelled HTLV-I cDNA probe but does not produce detectable amounts of HTLV-I p24 gag protein. $10^5$ ATH8 cells were pre-exposed to 2 $\mu$ g/ml polybrene for 30 minutes, pelleted, exposed to various amounts of HTLV-$III_B$, resuspended in 2 ml complete medium (RPMI supplemented with 15% undialysed, heat inactivated fetal calf serum, 4 mM L-glutamine, $5x10^{-5}$ 2-mercaptoethanol, 50 U/ml penicillin, and 50 $\mu$g/ml streptomycin) containing 15% (vol/vol)-interleukin2 (lectin-depleted; Cellular Products Inc., Buffalo, NY), and incubated in culture tubes (3033, Falcon, Oxnard, CA) at 37°C in 5% $CO_2$-containing humidified air. On day 7, the total viable cells were counted by the trypan blue dye exclusion method. Table 1 shows the cytopathic effect of HTLV-III/LAV on the ATH8 cells; data are expressed as the arithmetic means ±1 standard deviation for duplicate determinations.

Table 1

Cytopathic Effect of HTLV-III/LAV on ATH8 Cells

| Number of $HTLV-III_B$ Virus Particles/Cell | Number of Viable ATH8 Cells ($x10^5$) |
|---|---|
| 0 | 3.37 ± 0.1 |
| 0.05 | 3.36 ± 0.04 |
| 0.5 | 3.26 ± 0.15 |
| 5 | 1.97 ± 0.2 |
| 50 | 1.78 ± 0.16 |
| 500 | 0.37 ± 0.02 |
| 5,000 | 0.30 ± 0.01 |

Table 2

Effect of 2',3'-Dideoxynucleosides on the in vitro Immune Reactivity of Normal Lymphocytes

| Responder Cells | None | Adenosine[1] | | Guanosine[1] | | Inosine[1] | | None[4] | Cytidine[4] | | Thymidine[4] | |
|---|---|---|---|---|---|---|---|---|---|---|---|---|
| | | 10 | 100 | 10 | 100 | 10 | 100 | | 1 | 10 | 200 | 2,000 |
| Clone TM3 [2] | 75± 6 | 86± 8 | 67± 2 | 66± 5 | 77± 1 | 62±4 | 81±4 | 12±1 | 13±1 | 8±1 | 9±1 | 9±1 |
| PBM + PHA [3] | 310±39 | 289±18 | 314±37 | 350± 4 | 469± 9 | 298±20 | 355±37 | 82±1 | 101±1 | 86±3 | 86±6 | 77±8 |
| PBM + PWM [3] | 63± 2 | 63± 5 | 57± 3 | 73± 8 | 62± 5 | 61± 1 | 62± 5 | 24±1 | 23±3 | 15±3 | 31±1 | 31±1 |

1 - All nucleosides tested are of 2',3'-dideoxy-configuration. Concentrations (μM) of each 2',3'-dideoxynucleoside bring about virtually complete inhibition of the cytopathic effect of HTLV-III/LAV (Figures 1 and 2A) and the viral p24 expression (except 2',3'-dideoxythymidine; see Figure 2B). $^3$H-thymidine incorporation was used as an indicator of activation of responder cells, unless otherwise indicated.

2 - $5 \times 10^4$ normal helper/inducer TM3 cells were stimulated with tetanus toxoid (0.6 limiting flocculation units/ml) plus $10^5$ irradiated (4,000 rad) autologous peripheral blood mononuclear cells(PBM) as a source of accessory cells and cultured for 72 hours in the presence or absence of the 2',3'-dideoxynucleoside. After exposure to 0.5 μCi $^3$H-thymidine or 1 μCi $^3$H-uridine for the final 5 hours, cells were harvested onto glass fibers and the incorporated radioactivity was counted. Data are expressed as the arithmetic mean counts per minute ($\times 10^3$) ±1 standard deviation triplicate determinations.

3 - $10^6$ PBM from a healthy individual were stimulated with phytohaemagglutinin (PHA) or pokeweed mitogen (PWM) and cultured for 72 hours and treated as described above.

4 - $^3$H-uridine incorporation was used as an indicator of activation of the responder cells.

The pyrimidine base which is the subject of this invention comprises the base itself and the conventional mono- and triphosphates and where 2',3'-dideoxycytidine is noted in the specification and claims, the phosphates are also included in the definition.

## Claims

1. A method for alleviating the cytopathic effects of HTLV-III virus characterised by in vitro contacting of said HTLV-III virus with 2,3'-dideoxycytidine or a mono- or triphosphate thereof.

2. A method of alleviating the cytopathic effects of HTLV-III virus characterised by in vitro contacting of said HTLV-III virus within a human T-cell with 2',3'-dideoxycytidine or a mono- or triphosphate thereof.

3. The use in the preparation of a medicament for the treatment of AIDS or a related disease of which HTLV-III is the etiological agent, of a therapeutically effective amount of 2',3'-dideoxcytidine or a mono- or triphosphate thereof, said therapeutically effective amount being insufficient to inhibit proliferation or immune reactivity of normal T-cells.

## Patentansprüche

1. Verfahren zum Abschwächen der zytopathischen Wirkungen des HTLV-III-Virus, dadurch gekennzeichnet, daß das HTLV-III-Virus in Vitro mit 2',3'-Dideoxycytidin oder einem Mono- oder Triphosphat davon in Berührung gebracht wird.

2. Verfahren zum Abschwächen der zytopathischen Wirkungen des HTLV-III-Virus, dadurch gekennzeichnet, daß das HTLV-III-Virus in Vitro innerhalb einer menschlichen T-Zelle mit 2',3'Dideoxycytidin oder einem Mono- oder Triphosphat hiervon in Berührung gebracht wird.

3. Verwendung einer therapeutisch wirksamen Menge von 2',3'-Dideoxycitidin oder einem Mono- oder Triphosphat hiervon, dessen therapeutisch wirksame Menge zur Verhinderung einer Vermehrung oder Immunreaktion normaler T-Zellen nicht ausreicht, bei der Zubereitung eines Medikaments zur Behandlung von AIDS oder einer verwandten Krankheit, deren ätiologisches Agens HTLV-III ist.

## Revendications

1. Procédé pour affaiblir le pouvoir cytopathologique du virus HTLV-III, caractérisé en ce qu'il consiste à mettre en contact, in vitro, ledit virus HTLV-III avec la didésoxy-2',3'-cytidine ou avec un de ses dérivés mono- et triphosphate.

2. Procédé pour affaiblir le pouvoir cytopathologique du virus HTLV-III, caractérisé en ce qu'il consiste à mettre en contact, in vitro, ledit virus HTLV-III, sous la forme d'un lymphocyte-T porteur dudit virus HTLV-III, avec la didésoxy-2',3'-cytidine ou avec un de ses dérivés mono- et triphosphate.

3. Utilisation de la didésoxy-2',3'-cytidine ou de son dérivé mono- ou triphophate, en quantité suffisante pour préparer un médicament pour traiter le SIDA ou une maladie qui lui est associée et pour laquelle le virus HTLV-III est l'agent étiologique, ladite quantité étant insuffisante pour inhiber la prolifération ou l'immunoréactivité des lymphocytes-T normaux.

FIG.1

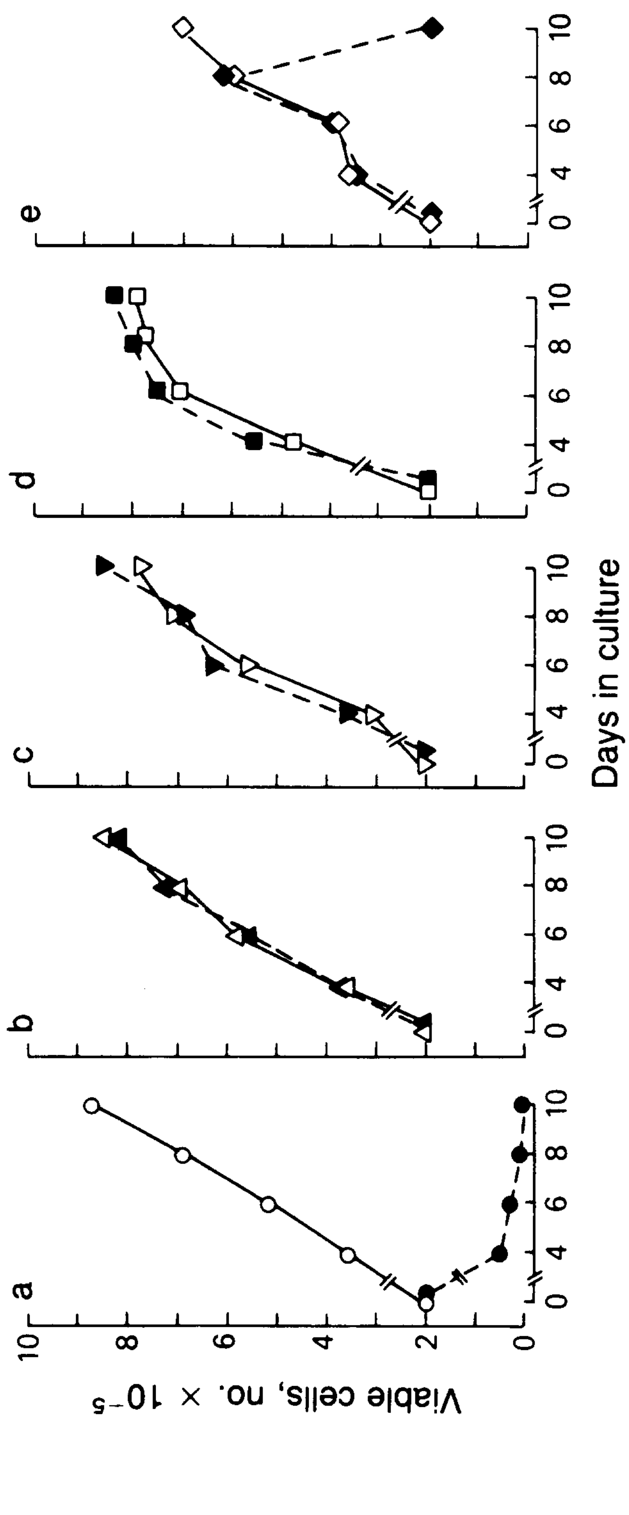

FIG.2A

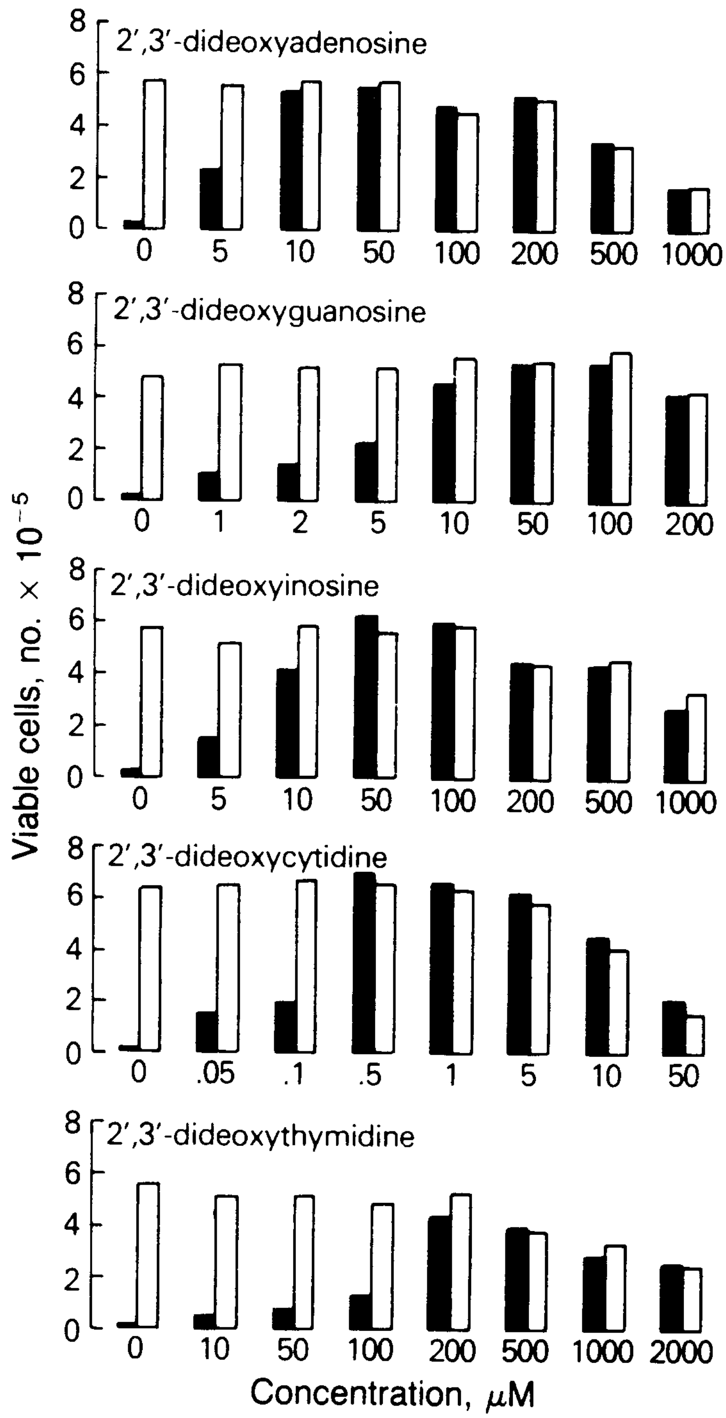
2',3'-dideoxyadenosine
0
5
10
50
100
200
500
1000
2',3'-dideoxyguanosine
0
1
2
5
10
50
100
200
2',3'-dideoxyinosine
0
5
10
50
100
200
500
1000
2',3'-dideoxycytidine
0
.05
.1
.5
1
5
10
50
2',3'-dideoxythymidine
0
10
50
100
200
500
1000
2000
Viable cells, no. × 10⁻⁵
8
6
4
2
0
Concentration, μM

FIG.2B

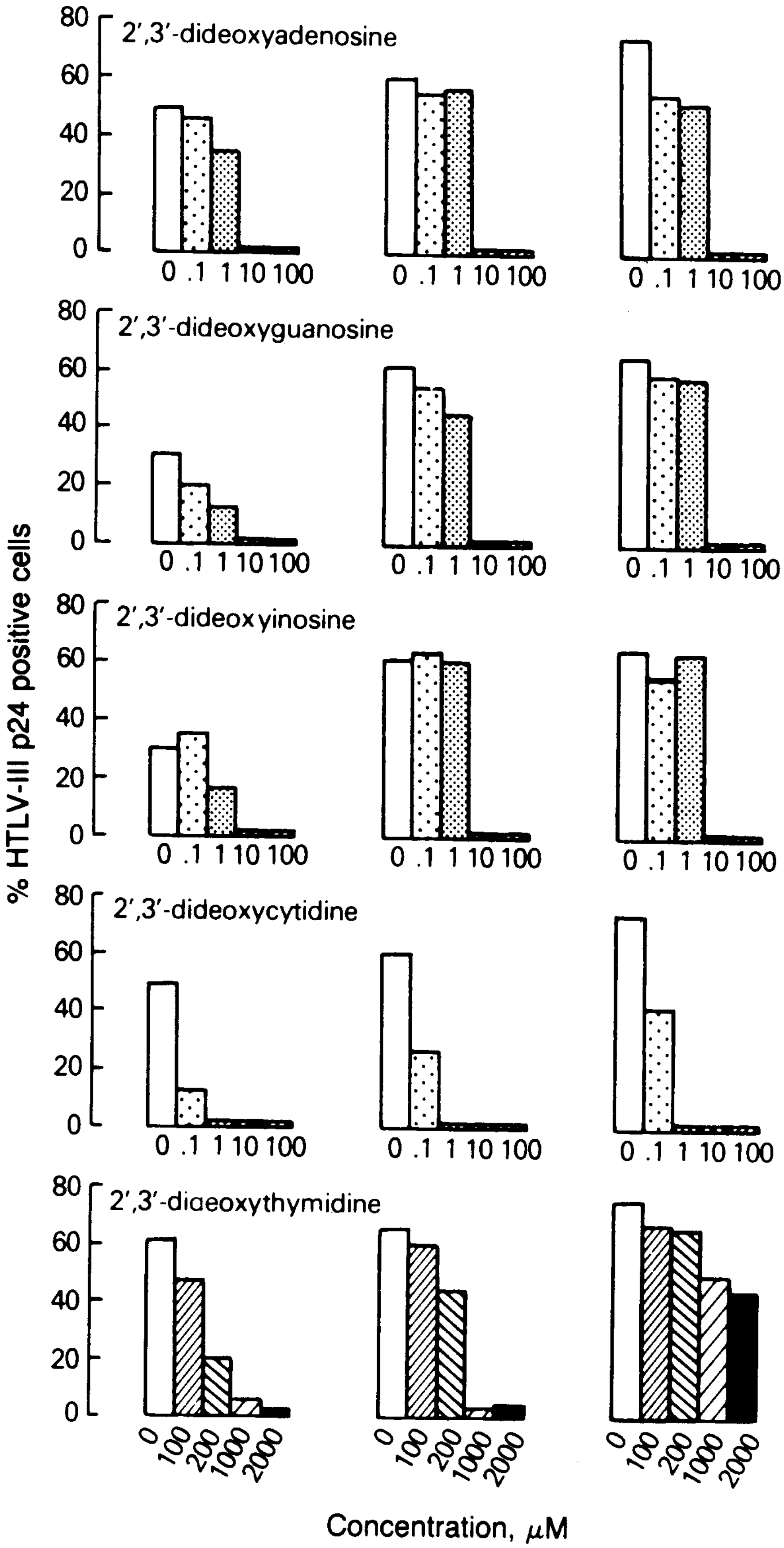
% HTLV-III p24 positive cells
80
60
40
20
0
2′,3′-dideoxyadenosine
2′,3′-dideoxyguanosine
2′,3′-dideoxyinosine
2′,3′-dideoxycytidine
2′,3′-diaeoxythymidine
0 .1 1 10 100
0
100
200
1000
2000
Concentration, μM

FIG.2C

a

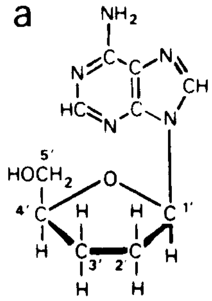

b

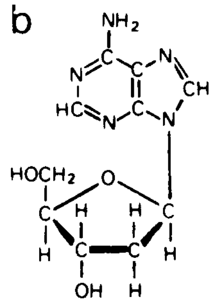

c

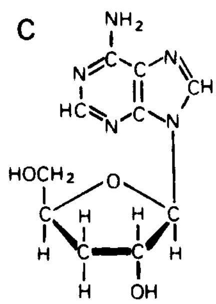

d

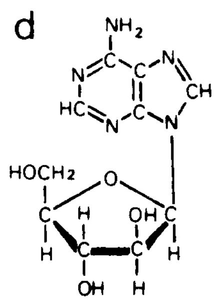

e